# EUROPEAN PATENT SPECIFICATION

(11) **EP 3 764 891 B1**
(45) Date of publication and mention of the grant of the patent: **11.02.2026**
(21) Application number: 19720178.3
(22) Date of filing: 18.03.2019
(51) Int. Cl.: A61B 5/369, A61B 5/055, A61B 5/245

(54) **METHOD OF DETERMINING BRAIN ACTIVITY**
VERFAHREN ZUR BESTIMMUNG DER HIRNAKTIVITÄT
PROCÉDÉ DE DÉTERMINATION DE L'ACTIVITÉ CÉRÉBRALE

(30) Priority: 16.03.2018 NL 2020601
(43) Date of publication of application: 20.01.2021
(73) Proprietor: Stichting VU, 1081 HV Amsterdam (NL)
(72) Inventor: LINKENKAER-HANSEN, Klaus, 1081 HV Amsterdam (NL); HARDSTONE, Richard Edward, 1081 HV Amsterdam (NL)
(74) Representative: V.O.
(86) International application number: PCT/NL2019/050167
(87) International publication number: WO 2019/177462

(56) References cited:
- WO-A1-2008/043365
- US-A1- 2009 048 530
- US-A1- 2017 065 199

## Description

### TECHNICAL FIELD

The various aspects and examples thereof relate to the field of processing data representing neural activity of a living being and brain activity of a human being in particular.

### BACKGROUND

Balanced excitation and inhibition is fundamental to brain function. A balanced excitation-inhibition ratio is crucial for proper functioning of neuronal circuits and is actively regulated by neuromodulation and maintained by mechanisms of homeostatic synaptic plasticity. Excitation and inhibition imbalances are seen as a common pathophysiological mechanism in many brain disorders. However, measurements of an excitation-inhibition ratio in animals or humans in vivo are difficult at this moment. In vitro measurements exist, but are cumbersome.

### SUMMARY

It is preferred to provide technology for determining an excitation-inhibition ratio that can be applied to data obtained from living beings like animals or humans.

A first aspect provides, in a device for processing biomedical data obtained from a body, a method of determining brain activity according to claim 1.

A correlation value is determined between the amplitude values and the corresponding variation values over at least a substantial part of the period of time; and the correlation value is provided as an output.

Experiments have shown that such amplitude increases as a ratio of excitation and inhibition neural activity of a part of a brain increases. Furthermore, the variation value of neural activity increases as the ratio increases if the ratio is inhibition dominant and decreases as the ratio increases if the ratio is excitation dominant.

Within the context of the description, oscillation signal data is to be understood as an AC (alternating current) part of a signal. Such oscillating signal or alternating component does not have to be (quasi-) harmonic or (quasi-) periodical to qualify as oscillating. It may have (pseudo)-random nature and the amplitude distribution within the frequency spectrum of the signal is not of particular relevance.

This novel understanding provides a basis for the subject-matter of this aspect. It is noted that the human brain is too complex to set a diagnosis based on the correlation alone. The correlation value, further processed or not, may provide a basis for a diagnosis by a medical practitioner, but does not provide a diagnosis. Setting a diagnosis for humans or for animals is not included in the scope of this aspect and other aspects. Likewise, the method discussed does not include any parts that may relate to invasion of the human body or an animal body.

The input data may be obtained by means of invasive or non-invasive measurement methods. Yet, any invasive data collection method itself falls outside the scope of this aspect and other aspects.

Prior to determining the correlation, various operations may be executed on the data collected. Such operations include, but are not limited to determining a variation value of an amplitude envelope or power envelope of the signal data, applying a low-, high- or bandpass filter to the signal data and determining a variation value of data after integrating or otherwise summing the signal data, detrending the signal data on a per timeslot basis after the summing and/or normalisation by the (average/median) amplitude in each timeslot and determining a variation of the detrended signal data, applying detrended fluctuation analysis on the signal data globally or per timeslot, obtaining a mean or median amplitude per timeslot as the amplitude value, other operations or a combination thereof.

Applying such operations remove certain features, for example of stochastic nature and amplify certain relevant features. This facilitates determining the correlation.

The first aspect further comprises determining the ratio of excitatory and inhibitory signalling of neurons to be inhibition dominated, if the correlation value is above a first threshold; determining the ratio of excitatory and inhibitory signalling of neurons to be excitation dominated if the correlation value is below a second threshold; and determining the ratio of excitatory and inhibitory signalling of neurons to be balanced if the correlation value is between the first and the second threshold.

The experimentally determined relation between the variation and the amplitude on one hand and the ratio excitation/inhibition activity of neurons in the brain, may further be used for determining a state of one or more brain regions. As indicated above, such determining does not constitute determining a pathological condition. Rather, it may serve a medical practitioner for setting a diagnosis. The first and the second threshold may be the same; the third condition is met if the correlation value is equal to that single threshold, optionally using a margin of 0.5%, 1%, 1.5%, 2%, 5%, 10%, 15% or another margin less than 20% and preferably less than 10%.

In another embodiment, the correlation value is used for providing an estimation of a ratio between excitatory and inhibitory signalling of neurons of at least a part of the brain from which the neuron oscillation signal data originates. Preferably, the value of the correlation is subtracted from one (1) and the resulting difference is used as an estimate. Experiments have shown this is at least a reasonable estimation.

A second aspect provides a computer program product according to claim 13.

A third aspect provides a device for processing biomedical data according to claim 14.

The time division module, the value determination module, the correlation module and, optionally, other data signal processing modules may be comprised in one or more general or dedicated processing modules that may be softwired by means or programming or hardwired by nonvolatile programming or manufacturing.

### BRIEF DESCRIPTION OF THE DRAWINGS

The various aspects and examples thereof will now be discussed in further detail. In the drawings,
Figure 1: shows a signal processing device 100 connected to an electro encephalogram signal acquiring device;
Figure 2: shows a flowchart; and
Figure 3 A through Figure 3 G show graphs of processed signal data.

### DETAILED DESCRIPTION

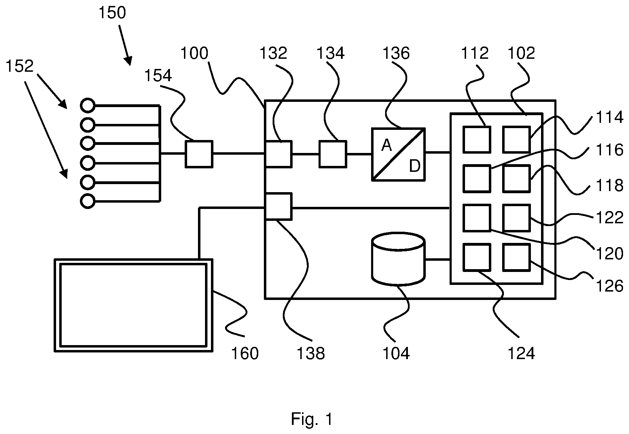

Figure 1 shows an electro encephalogram signal acquiring device 150, a signal processing device 100 and a display screen 160. The electro encephalogram signal acquiring device 150 comprises electrodes 152 that may be placed on the head of a subject under scrutiny and a human being in particular. The electro encephalogram signal acquiring device 150 further comprises a signal conditioning circuit 154. The signal conditioning circuit 154 is arranged for signal conditioning of signals received from the electrodes.

The conditioning may comprise amplification, filtering, transformation, other, or a combination thereof. The signal conditioning circuit 154 may also be arranged for providing a signal to the electrodes 152, which electrodes 152 may modify the provided signal. The modified signal is received by the signal conditioning circuit 154 and may be conditioned. the electrodes 152 may be provided internally or externally of the person under scrutiny.

The signal processing device comprises an input module 132, an optional filter module 134, an analogue to digital converter 136, a processing module 102, a storage module 104 and an output module 138. The input module 132 is arranged for receiving a signal from the electro encephalogram signal acquiring device 150. As such, it may comprise a plug or a socket. The filter module 134 may be arranged for filtering the received signal. The filter module 134 is arranged to pass through AC signal components of a wide frequency range (e.g., 0-20.000 Hz), preferably with no high-pass filtering. Optionally, the filter module 134 may be a bandpass filter in a more narrow frequency range, for example 0.1-500 Hz or any other band. Optionally, a DC signal may be passed through as well.

Such filtering may comprise low pass, band pass or high pass filtering, other filtering, or a combination thereof. The filtering may be active or passive filtering. The analogue to digital converter 136 is arranged for sampling the filtered signal and transforming it to the digital domain - with respect to time and amplitude. Alternatively, the analogue to digital converter 136 is provided upstream of the filter module 134. In that case, the filter module 134 is a digital filter; otherwise an analogue filter.

The processing module 102 comprises various submodules for processing the digitised signal. It is noted that, optionally, the signal processing may also be arranged for processing a signal in the analogue domain, but the digital domain is preferred. For processing the signal, the processing module 102 comprises a timeslot submodule 112 for assigning one or more consecutive samples to a timeslot or, worded differently, a time window.

The processing module 102 further comprises an amplitude analysis submodule 114 arranged for determining an amplitude envelope of a signal. Alternatively or additionally, a power envelope may be obtained. It is noted that power of a signal is directly related to the amplitude of the signal. Therefore, in this document, unless specified otherwise, a reference to amplitude of the acquired signal is equivalent to a reference to power of the acquired signal, either processed or not.

Furthermore, the processing module 102 comprises a signal integration submodule 116 as a summing submodule. In a preferred embodiment, the signal integration module is arranged to sum a demeaned signal. Such demeaned signal is obtained by subtracting from an actual signal value a mean or median value calculated over a particular timeframe. The processing module 102 also comprises a normalising submodule 118 for normalising a signal. Additionally, the processing module 102 comprises a detrending submodule 120 for detrending a signal.

The processing module 102 further comprises a variation determining submodule 122 for determining variation of a signal within a particular amount of time, like within a timeslot. The variation of a signal may be determined as the standard deviation, but determining the variation by other means may be envisaged as well. The processing module 102 also comprises a correlation submodule 124 for determining correlation between two datasets.

Furthermore, the processing module 102 comprises a comparison module for comparing a calculated or otherwise obtained value with a pre-determined value that may be stored in the storage module 104.

It is noted that in various examples, the processing module 102 may comprise one or more of the processing submodules discussed above, depending on various demands in particular scenarios; not all submodules are required for implementation of the various aspects.

The storage module 104 is arranged for storing results of processing signals by the processing module 102. Furthermore, the storage module 104 may, as for example a non-transitory medium, comprise computer executable code for programming the processing module 102 to carry out various methods for signal processing. And the storage module 104 may hold various values used for signal processing.

The screen 160 is connected to an output module 138 comprised by the signal processing device 100. As such, the screen 160 may be provided with processed data signals, either in values, in graphical representations, other or a combination thereof.

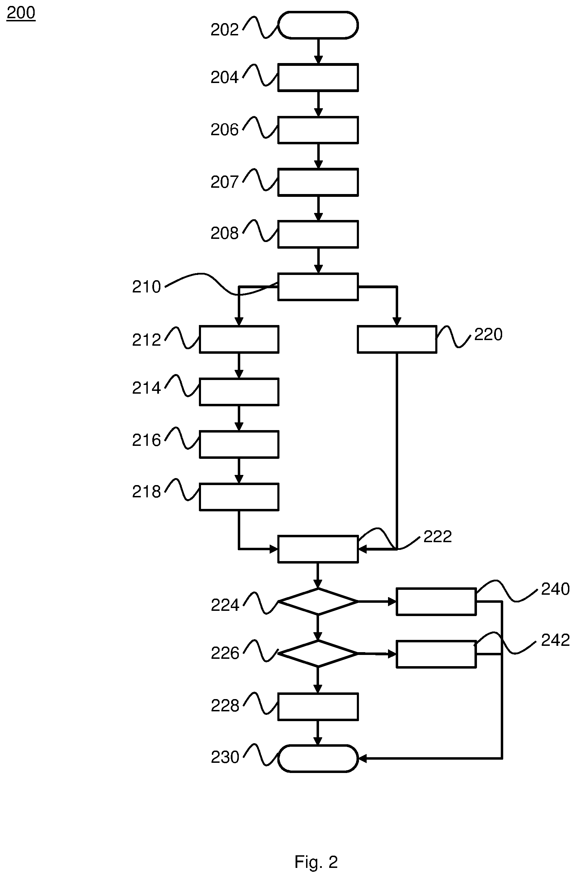

Figure 2 shows a flowchart 200 depicting a procedure for signal processing that may be carried out by the signal processing device 100 as shown by Figure 1. The various parts of the flowchart are briefly summarised in the list below. It is noted that whereas parts of the flowchart 200 may be referred to as steps, it is noted that processing parts indicated as one step may in other examples be distributed over multiple steps and the other way around. The various parts are summarised as follows:
- 202: start
- 204: obtain signal
- 206: sample signal
- 207: bandpass filter signal
- 208: assign samples to timeslots
- 210: determine amplitude envelope
- 212: sum values
- 214: normalise values
- 216: detrend values
- 218: determine variation of the values
- 220: determine mean amplitude
- 222: correlate variation and mean amplitude for slots
- 224: higher than first threshold?
- 226: lower than second threshold?
- 228: balance excitation and inhibition
- 230: end
- 240: inhibition dominant
- 242: excitation dominant

The process starts in a terminator 202 and proceeds to step 204 in which the signal processing device 100, or, in another example, the signal processing module 102 receives a signal comprising data representing brain activity. Such signal may be obtained from the electro encephalogram signal acquiring device 150, but also data from magneto encephalography or invasively recorded local field potentials may be used. Additionally or alternatively, data is acquired by means of single or multi-channel local field potentials, voltage sensitive dies, functional magnetic resonance imaging or near-infrared spectroscopy. The received signal is processed by means of one or more different operations. Each operation may be executed by an applicable submodule of the signal processing module 102. Not every operation is necessary and various operations may be omitted or combined. Furthermore, other operations may be added. The operations may be executed as shown by the flowchart 200 or in another order without departing from a general inventive concept.

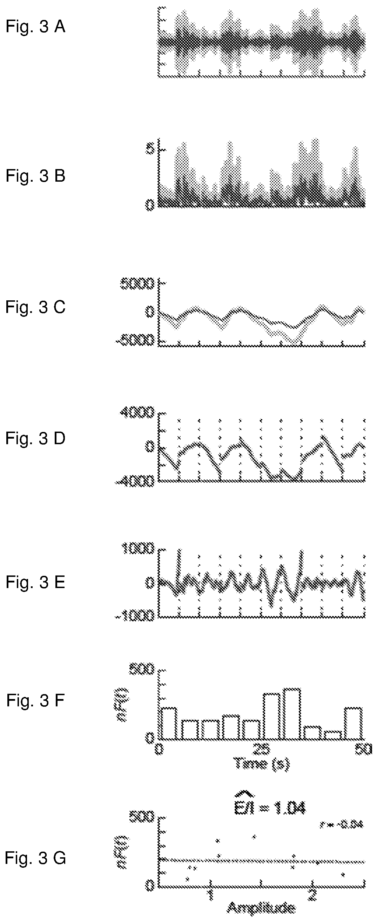

The process continues to step 206, in which the received signal is sampled and preferably also transformed to the digital domain with respect to the amplitude. The signal thus obtained is optionally provided to a further bandpass filter in step 207. The filtering of the further bandpass filter is preferably obtained by filtering in bands that are narrower than an optional filtering band of the bandpass filter 134. The pass band of the further bandpass filter may be adjustable providing for example a band from 1 Hz to 4 Hz, from 4 Hz to 8 Hz, from 8 Hz to 13 Hz and so on. The output of this operation is provided in Figure 3 A. Consecutive values of the digitised signal values are assigned to timeslots in step 208. Hence, one timeslot preferably comprises multiple signal values. Consecutive timeslots may overlap, such that at last some signal values may be assigned to two or more consecutive timeslots.

Of the various signal values, the amplitude envelope is subsequently determined in step 210. The output of this operation is provided in Figure 3 B. The amplitude envelope is determined over all signal values or at least a substantial part thereof, irrespective of the timeslots. The amplitude envelope may be determined by means of an absolute Hilbert transform, but also, additionally or alternatively, by means of other methods. Additionally or alternatively, an envelope of the power of the signal is determined.

Subsequently, the process splits in two branches. In the left branch, the signal envelope is integrated in step 212. Alternatively or additionally, signal values are summed in another way. Such summing may be preceded by a demeaning step as discussed above. The demeaning and/or the summing may be processed over the whole time period or over a timeslot. The output of this operation is provided in Figure 3 C. Such integration may be executed by simple arithmetic adding or specific integration algorithms. In another example, the signal is provided to a lowpass filter.

The values of the integrated signal are, on a per-timeslot basis, normalised in step 214. The normalisation may be executed based on an average value per timeslot. The output of this operation is provided in Figure 3 D. As this is preferably the first operation that is executed on a per-timeslot basis, the signal values may alternatively, also be assigned to timeslots between step 214 and step 216.

The signal values thus normalised are, on a per-timeslot basis, detrended in step 216. Various algorithms are available for detrending a signal. The output of this operation is provided in Figure 3 E.

Of the integrated, normalised and detrended values of the determined signal envelope, the variation is calculated in step 218. The output of this operation is provided in Figure 3 F. Such variation may be the standard deviation. Alternatively or additionally, also other algorithms for determining a variation of a parameter may be employed.

In the right branch, a mean or median amplitude or amplitude envelope is calculated for values assigned to a particular timeslot in step 220. Depending on the application, either operation - mean or median - may be calculated. In further examples, outliers may be removed from a dataset before calculating the mean or median value. Alternatively or additionally the mean or median of the power or power envelope may be calculated per timeslot.

In step 222, a correlation between the variation per timeslot and the mean value calculated in step 220 is determined over the consecutive timeslots in which the obtained signal is divided. The output of this operation is provided in Figure 3 G. The correlation value is subsequently compared to a first threshold value in step 224. The value of the threshold will be discussed below. If the correlation is lower than the first threshold, the procedure branches to the right and it is determined in step 240 that the ratio of excitation and inhibition signalling is inhibition dominant.

If the correlation value is lower than the first threshold, the procedure proceeds to step 226. In step 226, the correlation value is compared to a second threshold value. If the correlation value is lower than the second threshold, the process branches to the right and it is determined in step 242 that the ratio of excitation and inhibition is excitation dominant.

If the correlation value is higher than the first threshold and lower than the second threshold, the process continues to step 228 in which it is determined that the ratio of excitation and inhibition is balanced.

It has been determined that the correlation between the mean or median of the power or power envelope per slot and the variation of the signal and more in particular of the signal power or the signal power envelope (or signal amplitude envelop) provides an estimation of the ratio of excitation and inhibition in a part of the brain of which the signal has been obtained. For obtaining such correlation value, one or more operations on the obtained signal data may be required, as discussed above. With 1 minus the correlation being below 1, the ratio of excitation and inhibition is inhibition dominant. And with 1 minus the correlation being above 1, the ratio of excitation and inhibition is excitation dominant. Hence, such third threshold may be set at 1; if 1 minus the correlation has been determined to be substantially equal to 1, the ratio of excitation and inhibition is balanced. In such case, there is no correlation between the two datasets.

In case of using two threshold values, as depicted by Figure 2, the lower first threshold may be set at 0.99, 0.95, 0.9, 0.85 or 0.8 and the higher second threshold may be set at 1.01, 1.05, 1.1, 1.15 or 1.2. Alternatively or additionally, not the value of 1 minus the correlation is assessed, but the correlation may be assed. In such case, the lower first threshold may be set at -0.01, -0.05, -0.1, -0.15 or -0.2 and the higher second threshold may be set at 0.01, 0.05, 0.1, 0.15 or 0.2.

Having determined the ratio of excitation and inhibition signalling in a population of neurons, the process ends in a terminator 230.

In an alternative implementation, further factors may be taken into account for determining whether the ratio of excitation and inhibition is either inhibition or excitation dominated - or balanced. For example, the DFA value may be taken into account. If the DFA is found to be less than a pre-determined DFA value and the received signal has no major oscillations, i.e. the median amplitude or power envelope is relative low, it may be determined that the ratio of excitation and inhibition is inhibition dominant. For such determination, the pre-determined DFA value may be set at any value between 0.5 and 0.65 and preferably at 0.6. If the received signal is determined to oscillate significantly, the ratio of excitation and inhibition is determined to be excitation dominant. Alternatively, as an option, if the signal is smaller than the pre-determined DFA value, the ratio of excitation to inhibition is not estimated.

In the description above, it will be understood that when an element such as layer, region or substrate is referred to as being "on" or "onto" another element, the element is either directly on the other element, or intervening elements may also be present. Also, it will be understood that the values given in the description above, are given by way of example and that other values may be possible and/or may be strived for.

Furthermore, it is also possible to use less components than described here, wherein one component carries out multiple functions. Just as well, it is also possible to use more elements than depicted in the Figures, wherein functions carried out by one component are distributed over multiple components.

It is to be noted that the figures are only schematic representations that are given by way of non-limiting examples. The word 'comprising' does not exclude the presence of other features or steps than those listed in a claim. Furthermore, the words 'a' and 'an' shall not be construed as limited to 'only one', but instead are used to mean 'at least one', and do not exclude a plurality.

It is stipulated that the reference signs in the claims do not limit the scope of the claims, but are merely inserted to enhance the legibility of the claims.

## Claims

1. In a device for processing biomedical data obtained from a body, a method of determining brain activity, comprising:
- Obtaining (204) neuron oscillation signal data of at least a part of a brain over a period of time;
- Apportioning (208) the neuron oscillation signal data in timeslots within the period;
- Per time slot, based on the neuron oscillation signal data for the timeslot, obtaining an amplitude value and variation value representing the signal data values in the timeslot;
- Determining (222) a correlation value between the amplitude values and the corresponding variation values over at least a substantial part of the time period;
- Providing the correlation value as an output; and
**characterised in that** the method further comprises:
- If the correlation value is above a first threshold, determining (240) the ratio of excitatory and inhibitory signalling of neurons to be inhibitory dominated;
- If the correlation value is below a second threshold, determining (242) the ratio of excitatory and inhibitory signalling of neurons to be excitatory dominated; and
- If the correlation value is between the first and the second threshold, determining (228) the ratio of excitatory and inhibitory signalling of neurons to be balanced.

2. Method according to claim **1,** wherein obtaining the variation value comprises determining a variation value of an amplitude envelope or power envelope of the signal data.

3. Method according to claim 1 or 2, wherein obtaining the variation value further comprises calculating, on a per data value basis, a cumulative sum based on amplitude envelope data values and determining a variation value of data after calculation of the cumulative sum.

4. Method according to claim 3, further comprising:
- obtaining a mean or median value of the neuron oscillation signal data;
- subtracting the mean or median value from momentary signal values; and
- summing of the resulting values.

5. Method according to claim 3 or 4, wherein obtaining the variation value further comprises detrending the signal data on a per timeslot basis after application of the summation and determining a variation of the detrended signal data.

6. Method according to any of the preceding claims, wherein obtaining the variation value comprises applying detrended fluctuation analysis on the signal data per timeslot.

7. Method according to any of the preceding claims, wherein obtaining an amplitude value comprises obtaining a mean amplitude of the neuron oscillation signal per timeslot as the amplitude value.

8. Method according to any of the preceding claims, wherein obtaining an amplitude value comprises obtaining a median amplitude of the neuron oscillation signal per timeslot as the amplitude value.

9. Method according to any of the claims 1-6, wherein obtaining an amplitude value comprises:
- obtaining an amplitude envelope for the signal data; and
- obtaining a mean value or a median value of the amplitude envelope as the amplitude value.

10. Method according to the preceding claims, wherein the timeslots overlap.

11. Method according to any of the preceding claims, wherein the neuron oscillation signal data is obtained by at least one of:
- electro encephalography;
- magneto encephalography;
- single or multi-channel local field potentials;
- voltage sensitive dies;
- functional magnetic resonance imaging; and
- near-infrared spectroscopy.

12. Method according to any of the preceding claims, providing an estimation of a ratio between excitatory and inhibitory signalling of neurons of at least a part of the brain from which the neuron oscillation signal data originates, based on the correlation value.

13. Computer program product comprising computer executable code for programming a computer to enable the computer to execute a method according to any of the preceding claims.

14. Device for processing biomedical data obtained from a body, the device comprising:
- an input module (132) arranged to obtaining neuron oscillation signal data of a at least a part of a brain over a period of time;
- a time division module (112) arranged to apportion oscillation data in timeslots within the period;
- a value determination module arranged to, per time slot, based on the oscillation data for the timeslot, obtain an amplitude value and variation value representing the signal data values in the timeslot;
- a correlation module (124) arranged to determining a correlation value between the amplitude values and the corresponding variation values over the first time period; and
- an output arranged to providing the correlation value; and
**characterised in that** the device is further arranged to:
- If the correlation value is above a first threshold, determine the ratio of excitatory and inhibitory signalling of neurons to be inhibitory dominated;
- If the correlation value is below a second threshold, determine the ratio of excitatory and inhibitory signalling of neurons to be excitatory dominated; and
- If the correlation value is between the first and the second threshold, determine the ratio of excitatory and inhibitory signalling of neurons to be balanced.

## Patentansprüche

1. Verfahren zur Bestimmung der Hirnaktivität in einer Vorrichtung zur Verarbeitung biomedizinischer Daten, die aus einem Körper gewonnen werden, umfassend:
- Gewinnen (204) von Neuronen-Oszillationssignaldaten mindestens eines Teils eines Gehirns über einen Zeitraum;
- Aufteilen (208) der Neuronen-Oszillationssignaldaten in Zeitabschnitte innerhalb des Zeitraums;
- Erfassen eines Amplitudenwerts und eines Variationswerts pro Zeitabschnitt, die die Signaldatenwerte in dem Zeitabschnitt darstellen, basierend auf den neuronalen Oszillationssignaldaten für den Zeitabschnitt;
- Bestimmen (222) eines Korrelationswerts zwischen den Amplitudenwerten und den entsprechenden Variationswerten über mindestens einen wesentlichen Teil des Zeitraums;
- Bereitstellen des Korrelationswerts als Ausgabe; und
**dadurch gekennzeichnet, dass** das Verfahren ferner umfasst:
- Wenn der Korrelationswert über einem ersten Schwellenwert liegt, Bestimmen (240) des Verhältnisses von exzitatorischer und inhibitorischer Signalübertragung von Neuronen als inhibitorisch dominiert;
- Wenn der Korrelationswert unter einem zweiten Schwellenwert liegt, Bestimmen (242) des Verhältnisses von exzitatorischer und inhibitorischer Signalübertragung von Neuronen als exzitatorisch dominiert; und
- wenn der Korrelationswert zwischen dem ersten und dem zweiten Schwellenwert liegt, Bestimmen (228) des Verhältnisses von exzitatorischer und inhibitorischer Signalübertragung von Neuronen als ausgeglichen.

2. Verfahren nach Anspruch 1, wobei das Ermitteln des Variationswerts das Bestimmen eines Variationswerts einer Amplitudenhüllkurve oder einer Leistungshüllkurve der Signaldaten umfasst.

3. Verfahren nach Anspruch 1 oder 2, wobei das Ermitteln des Variationswerts ferner das Berechnen einer kumulativen Summe auf Basis von Amplitudenhüllkurven-Datenwerten pro Datenwert und das Bestimmen eines Variationswerts der Daten nach dem Berechnen der kumulativen Summe umfasst.

4. Verfahren nach Anspruch 3, ferner umfassend:
- Ermitteln eines Mittel- oder Medianwerts der Neuronen-Oszillationssignaldaten;
- Subtrahieren des Mittel- oder Medianwerts von momentanen Signalwerten; und
- Summenbildung der resultierenden Werte.

5. Verfahren nach Anspruch 3 oder 4, wobei das Ermitteln des Variationswerts ferner die Trendbereinigung der Signaldaten auf einer Pro-Zeitabschnitt-Basis nach Anwendung der Summenbildung und das Bestimmen einer Variation der trendbereinigten Signaldaten umfasst.

6. Verfahren nach einem der vorstehenden Ansprüche, wobei das Ermitteln des Variationswerts das Anwenden einer trendbereinigten Fluktuationsanalyse auf die Signaldaten pro Zeitabschnitt umfasst.

7. Verfahren nach einem der vorstehenden Ansprüche, wobei das Ermitteln eines Amplitudenwerts das Ermitteln einer mittleren Amplitude des Neuronen-Oszillationssignals pro Zeitabschnitt als Amplitudenwert umfasst.

8. Verfahren nach einem der vorstehenden Ansprüche, wobei das Ermitteln eines Amplitudenwerts das Ermitteln einer medianen Amplitude des Neuronen-Oszillationssignals pro Zeitabschnitt als Amplitudenwert umfasst.

9. Verfahren nach einem der Ansprüche 1 bis 6, wobei das Ermitteln eines Amplitudenwerts umfasst:
- das Ermitteln einer Amplitudenhüllkurve für die Signaldaten; und
- das Ermitteln eines Mittelwerts oder eines Medianwerts der Amplitudenhüllkurve als Amplitudenwert.

10. Verfahren nach den vorstehenden Ansprüchen, wobei sich die Zeitabschnitte überlappen.

11. Verfahren nach einem der vorstehenden Ansprüche, wobei die Neuronenoszillationssignaldaten durch mindestens eines der folgenden Verfahren ermittelt werden:
- Elektroenzephalographie;
- Magnetenzephalographie;
- ein- oder mehrkanalige lokale Feldpotenziale;
- spannungsempfindliche Chips;
- funktionelle Magnetresonanztomographie; und
- Nahinfrarotspektroskopie.

12. Verfahren nach einem der vorstehenden Ansprüche, das eine Schätzung eines Verhältnisses zwischen exzitatorischen und inhibitorischen Signalen von Neuronen mindestens eines Teils des Gehirns, aus dem die Neuronenoszillationssignaldaten stammen, auf der Grundlage des Korrelationswerts liefert.

13. Computerprogrammprodukt, das einen computergestützten ausführbaren Code zum Programmieren eines Computers umfasst, damit der Computer ein Verfahren nach einem der vorstehenden Ansprüche ausführen kann.

14. Vorrichtung zum Verarbeiten von aus einem Körper gewonnenen biomedizinischen Daten, wobei die Vorrichtung umfasst:
- ein Eingabemodul (132), das zum Erfassen von Neuronen-Oszillationssignaldaten eines mindestens einen Teils eines Gehirns über einen Zeitraum angeordnet ist;
- ein Zeitaufteilungsmodul (112), das so ausgelegt ist, dass es Oszillationsdaten in Zeitabschnitte innerhalb des Zeitraums aufteilt;
- ein Wertbestimmungsmodul, das so ausgelegt ist, dass es pro Zeitabschnitt auf der Grundlage der Oszillationsdaten für den Zeitabschnitt einen Amplitudenwert und einen Variationswert erhält, die die Signaldatenwerte im Zeitabschnitt darstellen;
- ein Korrelationsmodul (124), das so ausgelegt ist, dass es einen Korrelationswert zwischen den Amplitudenwerten und den entsprechenden Variationswerten über den ersten Zeitraum ermittelt; und
- eine Ausgabe, die so ausgelegt ist, dass sie den Korrelationswert bereitstellt; und
**dadurch gekennzeichnet, dass** die Vorrichtung ferner so ausgelegt ist, dass sie Folgendes bewirkt:
- Wenn der Korrelationswert über einem ersten Schwellenwert liegt, bestimmt sie das Verhältnis von exzitatorischer und inhibitorischer Signalübertragung von Neuronen als inhibitorisch dominiert;
- wenn der Korrelationswert unter einem zweiten Schwellenwert liegt, bestimmt sie das Verhältnis von exzitatorischer und inhibitorischer Signalübertragung von Neuronen als exzitatorisch dominiert; und
- wenn der Korrelationswert zwischen dem ersten und dem zweiten Schwellenwert liegt, bestimmt sie das Verhältnis von exzitatorischer und inhibitorischer Signalübertragung von Neuronen als ausgeglichen.

## Revendications

1. Dans un dispositif de traitement de données biomédicales obtenues à partir d'un corps, procédé de détermination de l'activité cérébrale, comprenant :
- l'obtention (204) de données de signal d'oscillation neuronale d'au moins une partie d'un cerveau sur une période ;
- la répartition (208) des données de signal d'oscillation neuronale en intervalles de temps sur la période ;
- par intervalle de temps, sur la base des données de signal d'oscillation neuronale pour l'intervalle de temps, l'obtention d'une valeur d'amplitude et d'une valeur de variation qui représentent les valeurs des données de signal dans l'intervalle de temps ;
- la détermination (222) d'une valeur de corrélation entre les valeurs d'amplitude et les valeurs de variation correspondantes sur au moins une partie substantielle de la période ;
- la restitution de la valeur de corrélation comme un résultat ; et
**caractérisé en ce que** le procédé comprend en outre :
- si la valeur de corrélation est supérieure à un premier seuil, la détermination (240) du rapport de signalisation excitatrice et inhibitrice des neurones qui doivent être dominés de manière inhibitrice ;
- si la valeur de corrélation est inférieure à un deuxième seuil, la détermination (242) du rapport de signalisation excitatrice et inhibitrice des neurones qui doivent être dominés de manière excitatrice ; et
- si la valeur de corrélation se situe entre le premier et le deuxième seuils, la détermination (228) du rapport de signalisation excitatrice et inhibitrice des neurones à équilibrer.

2. Procédé selon la revendication 1, dans lequel l'obtention de la valeur de variation comprend la détermination d'une valeur de variation d'une enveloppe d'amplitude ou d'une enveloppe de puissance des données de signal.

3. Procédé selon la revendication 1 ou 2, dans lequel l'obtention de la valeur de variation comprend en outre le calcul, par valeur de données, d'une somme cumulative sur la base des valeurs de données d'enveloppe d'amplitude et la détermination d'une valeur de variation des données après le calcul de la somme cumulative.

4. Procédé selon la revendication 3, comprenant en outre :
- l'obtention d'une valeur moyenne ou médiane des données de signal d'oscillation neuronale ;
- la soustraction de la valeur moyenne ou médiane des valeurs de signal momentanées ; et
- la somme des valeurs résultantes.

5. Procédé selon la revendication 3 ou 4, dans lequel l'obtention de la valeur de variation comprend en outre la décomposition des données de signal par intervalle de temps après l'application de la sommation et la détermination d'une variation des données de signal décomposées.

6. Procédé selon l'une quelconque des revendications précédentes, dans lequel
l'obtention de la valeur de variation comprend l'application d'une analyse de fluctuation décomposée aux données de signal par intervalle de temps.

7. Procédé selon l'une quelconque des revendications précédentes, dans lequel
l'obtention d'une valeur d'amplitude comprend l'obtention d'une amplitude moyenne du signal d'oscillation neuronale par intervalle de temps en guise de valeur d'amplitude.

8. Procédé selon l'une quelconque des revendications précédentes, dans lequel
l'obtention d'une valeur d'amplitude comprend l'obtention d'une amplitude médiane du signal d'oscillation neuronale par intervalle de temps en guise de valeur d'amplitude.

9. Procédé selon l'une quelconque des revendications 1 à 6, dans lequel l'obtention d'une valeur d'amplitude comprend :
- l'obtention d'une enveloppe d'amplitude pour les données de signal ; et
- l'obtention d'une valeur moyenne ou d'une valeur médiane de l'enveloppe d'amplitude en guise de valeur d'amplitude.

10. Procédé selon les revendications précédentes, dans lequel les intervalles de temps se chevauchent.

11. Procédé selon l'une quelconque des revendications précédentes, dans lequel les données de signal d'oscillation neuronale sont obtenues par au moins l'un :
- d'une électroencéphalographie ;
- d'une magnétoencéphalographie ;
- de potentiels de champs locaux mono ou multicanal ;
- de colorants sensibles à la tension ;
- d'une imagerie à résonance magnétique fonctionnelle ; et
- d'une spectroscopie en proche infrarouge.

12. Procédé selon l'une quelconque des revendications précédentes, qui prévoit une estimation d'un rapport entre la signalisation excitatrice et inhibitrice des neurones d'au moins une partie du cerveau dont proviennent les données de signal d'oscillation neuronale, sur la base de la valeur de corrélation.

13. Produit de programme informatique comprenant un code exécutable par un ordinateur destiné à programmer un ordinateur afin de permettre à l'ordinateur d'exécuter un procédé selon l'une quelconque des revendications précédentes.

14. Dispositif de traitement de données biomédicales obtenues à partir d'un corps, le dispositif comprenant :
- un module d'entrée (132) prévu pour obtenir les données de signal d'oscillation neuronale d'au moins une partie d'un cerveau sur une période ;
- un module de division du temps (112) prévu pour répartir les données d'oscillation en intervalles de temps sur la période ;
- un module de détermination de valeur prévu pour, par intervalle de temps, sur la base des données d'oscillation pour l'intervalle de temps, obtenir une valeur d'amplitude et une valeur de variation qui représentent les valeurs de données de signal dans l'intervalle de temps ;
- un module de corrélation (124) prévu pour déterminer une valeur de corrélation entre les valeurs d'amplitude et les valeurs de variation correspondantes sur la première période ; et
- une sortie prévue pour fournir la valeur de corrélation ; et
**caractérisé en ce que** le dispositif est en outre prévu pour :
- si la valeur de corrélation est supérieure à un premier seuil, déterminer le rapport de signalisation excitatrice et inhibitrice des neurones qui doivent être dominés de manière inhibitrice ;
- si la valeur de corrélation est inférieure à un deuxième seuil, déterminer le rapport de signalisation excitatrice et inhibitrice des neurones qui doivent être dominés de manière excitatrice ; et
- si la valeur de corrélation se situe entre le premier et le deuxième seuils, déterminer le rapport de signalisation excitatrice et inhibitrice des neurones à équilibrer.
